# EUROPÄISCHE PATENTSCHRIFT

(11) **EP 0 510 218 B1**
(45) Veröffentlichungstag und Bekanntmachung des Hinweises auf die Patenterteilung: **15.02.1995**
(21) Anmeldenummer: 91106500.1
(22) Anmeldetag: 23.04.1991
(51) Int. Cl.: D06F 31/00

(54) **Verfahren und Vorrichtung zum Waschen und Desinfizieren in einer Durchlaufwaschmaschine**
Method and device for washing and disinfecting in a continuous batch washing machine
Procédé et dispositif pour laver et désinfecter dans une machine à laver type tunnel

(43) Veröffentlichungstag der Anmeldung: 28.10.1992
(73) Patentinhaber: Henkel-Ecolab GmbH & Co. OHG, 40554 Düsseldorf (DE)
(72) Erfinder: Herbertz, Heinz, W-8600 Bamberg (DE)

(56) Entgegenhaltungen:
- DE-A- 3 040 377
- DE-A- 3 935 621
- FR-A- 2 589 895

## Beschreibung

Die Erfindung betrifft ein Verfahren und eine Vorrichtung zum Waschen und Desinfizieren in einer Durchlaufwaschmaschine bei welchem die Wäsche durch eine Vorwaschzone, eine Klarwaschzone und eine Spülzone gefördert und Flotten aus der Klarwasch- und/oder der Spülzone zum Vorwaschen verwendet werden.

Zum Bleichen und Desinfizieren werden Hypochlorit und sauerstoffabspaltende Salze zunehmend durch Peressigsäure ersetzt, weil Chlorverbindungen im Abwasser unerwünscht und sauerstoffabspaltende Salze erst bei höheren Temperaturen wirksam werden.

Wasch- und Desinfektionsverfahren, wie in der Offenlegungsschrift DE 3040377 beschrieben, bei denen Flotten zurückgewonnen und zum Vorwaschen verwendet werden, können mit Peressigsäure nicht ohne weiteres durchgeführt werden. Da sich der Aktivsauerstoff bei diesem Mittel nur sehr langsam abbaut würden beim Vorwaschen mit zurückgewonnenen Flotten Verschmutzungen in der Wäsche fixiert, bevor sie ausgewaschen werden. Deshalb ist es üblich geworden, größere Mengen Frischwasser in den Vorwaschbereich zu leiten oder den Aktivsauerstoff mit Natriumbisulfit abzubauen. Dadurch steigt entweder der Wasserverbrauch oder die Salzfracht im Abwasser.

Der Erfindung liegt die Aufgabe zugrunde diese Probleme in ökonomisch und ökologisch sinnvoller Weise zu lösen.

Die Aufgabe wird erfindungsgemäß dadurch gelöst, daß in die zum Vorwaschen bestimmte Flotten ein Gas eingeleitet wird, das eine Verbindung mit Aktivsauerstoff eingeht. Bei der Vorrichtung zur Durchführung des Verfahrens wird das Gas über eine Kreiselpumpe eingeleitet.

Die Einleitung eines wirksamen Gases in die Waschflotten, anstatt pulverförmiger oder flüssiger Stoffe bringt eine schnellere Verteilung, Auflösung und Reaktion mit sich und keine Trägerstoffe belasten Transportmittel und Abwässer.

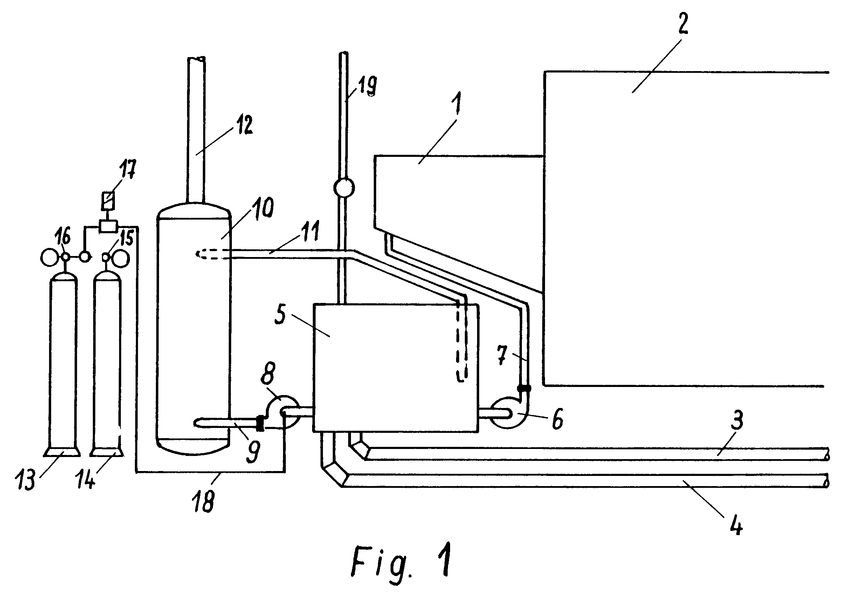

Ein Ausführungsbeispiel ist in Fig. 1 dargestellt und zeigt den Fülltrichter 1 zum Einbringen der Schmutzwäsche in die Vorwaschzone 2 der Durchlaufwaschmaschine. Über eine Rohrleitung 3 wird Flotte aus dem Spül- und Waschbereich und über eine Rohrleitung 4, Flotte aus dem Entwässerungsbereich in ein Sammelgefäß 5 geleitet und mit einer Pumpe 6 über eine Rohrleitung 7 zum Einweichen der Schmutzwäsche in den Fülltrichter 1 und in die Vorwaschzone 2 gefördert. Die Pumpe 8 fördert die Flotten aus dem Sammelgefäß 5 in den Behälter 10, dessen Einlaufstutzen 9 und Auslaufrohr 11 tangential und gegenüberliegend angebracht sind. Der Behälter 10 hat oben einen Abluftanschluß 12.

Das Gas strömt aus druckfesten Behältern (13, 14) durch Druckminderventile (15, 16) über ein Magnetventil 17 und eine Leitung 18 in die Pumpe 8. Zum Behälter 5 führt eine Frischwasserleitung 19.

Die Wirkungsweise ist folgende:
Bei Inbetriebnahme werden die Durchlaufwaschmaschine und der Behälter 5 mit Frischwasser gefüllt und Waschmittelstammlösung hinzugefügt. Die Schmutzwäscheposten fallen in den Fülltrichter 1 und werden mit Flotte aus dem Behälter 5 in die Vorwaschzone geschwemmt. Die Wäscheposten durchlaufen die Vorwaschzone, die Klarwaschzone, die Spülzone und den Entwässerungsbereich, wobei Waschmittel, Wasser Bleich- und Desinfektionsmittel, z. B. Peressigsäure, zugegeben werden. Ein Teil der gebrauchten Flotten wird zur Wiederverwendung in den Behälter 5 geleitet. Dann wird die Pumpe 8 eingeschaltet, das Magnetventil 17 geöffnet und das Gas, z.B. Ammoniak, strömt durch die Leitung 18 in die Pumpe 8. Dort wird das Gas mit der sehr viel größeren Flottenmenge gleichmäßig vermischt und durch den Einlaufstutzen 9 tangential in den vorzugsweise zylindrischen Behälter 10 geleitet, wodurch eine kreisende Bewegung der Flotten entsteht, die eine weitere Vermischung bewirkt und die gewünschte chemische Reaktion auslöst. Z.B. wird Aktivsauerstoff durch Ammoniak in Wasser und Stickstoff umgewandelt und letzterer durch den Abluftanschluß 12 abgeleitet.

## Patentansprüche

1. Verfahren zum Waschen und Desinfizieren in einer Durchlaufwaschmaschine, bei welchem die Wäsche durch eine Vorwaschzone, eine Klarwaschzone und eine Spülzone gefördert und Flotten aus der Klarwasch- und/oder der Spülzone zum Vorwaschen verwendet werden, dadurch gekennzeichnet, daß in die zum Vorwaschen bestimmten Flotten ein Gas eingeleitet wird, welches eine Verbindung mit Aktivsauerstoff eingeht.

2. Verfahren nach Anspruch 1, dadurch gekennzeichnet, daß Ammoniak in die zum Vorwaschen bestimmten Flotten eingeleitet wird.

3. Vorrichtung zur Durchführung des Verfahrens nach Anspruch 1, dadurch gekennzeichnet,daß das Gas über eine Kreiselpumpe (8) in die zum Vorwaschen bestimmten Flotten eingeleitet wird.

4. Vorrichtung nach Anspruch 3, dadurch gekennzeichnet, daß an einer Leitung (18) zur Kreiselpumpe (8) ein Anschluß mit Druckminderer für Gasbehälter (13, 14) angebracht ist.

5. Vorrichtung nach Anspruch 3, dadurch gekennzeichnet, daß die zum Vorwaschen bestimmten Flotten in einen Behälter (10) gepumpt werden, der einen Abluftanschluß (12) besitzt.

6. Vorrichtung nach Anspruch 3 und 4, dadurch gekennzeichnet, daß ein Sammelgefäß (5) die zum Vorwaschen bestimmten Flotten aufnimmt, die Kreiselpumpe (8) diese tangential in den Behälter (10) einleitet und von diesem in ständigem Kreislauf zum Sammelgefäß zurückgeführt werden, wobei das Gas injiziert wird.

## Claims

1. A washing and disinfecting process for a continuous batch washing machine in which the washing is transported through a prewash zone, a final wash zone and a rinse zone and liquors from the final wash zone and/or the rinse zone are used for prewashing, characterized in that a gas which combines with active oxygen is introduced into the liquors intended for prewashing.

2. A process as claimed in claim 1, characterized in that ammonia is introduced into the liquors intended for prewashing.

3. An arrangement for carrying out the process claimed in claim 1, characterized in that the gas is introduced into the liquors intended for prewashing via a rotary pump (8).

4. An arrangement as claimed in claim 3, characterized in that a connection with a pressure relief valve for gas containers (13,14) is provided on a pipe (18) leading to the rotary pump (8).

5. An arrangement as claimed in claim 3, characterized in that the liquors intended for prewashing are pumped into a vessel (10) which has a waste air outlet (12).

6. An arrangement as claimed in claims 3 and 4, characterized in that a collecting tank (5) accommodates the liquors intended for prewashing, the rotary pump (8) introduces the liquors tangentially into the vessel (10) and the liquors are continuously returned to the collecting tank, the gas being injected during their return.

## Revendications

1. Procédé et dispositif pour laver et désinfecter dans une machine à laver de type tunnel où le textile passe dans une zone de prélavage, une zone de lavage et une zone de rinçage et on utilise les eaux de la zone de lavage et/ou de rinçage du prélavage, caractérisé en ce qu'on introduit dans les eaux destinées au prélavage, un gaz, qui subit une réaction avec l'oxygène actif.

2. Procédé selon la revendication 1, caractérisé en ce qu'on introduit l'ammoniac dans les eaux destinées au prélavage.

3. Dispositif de réalisation du procédé selon la revendication 1, caractérisé en ce qu'on introduit le gaz dans les eaux destinées au prélavage au moyen d'une pompe centrifuge (8).

4. Dispositif selon la revendication 3, caractérisé en ce que sur une conduite (18) aboutissant à la pompe centrifuge (8) est branché un détendeur en aval des bouteilles de gaz (13, 14).

5. Dispositif selon la revendication 3, caractérisé en ce qu'on pompe les eaux destinées au prélavage dans une cuve (10), qui possède un évent (12).

6. Dispositif selon les revendications 3 et 4, caractérisé en ce qu'un bac de rétention (5) recueille les eaux destinées au prélavage, la pompe centrifuge (8) les introduit tangentiellement dans la cuve (10) et de là elles sont conduites en recyclage permanent dans le bac de rétention, tout en subissant l'injection de gaz.
